# EUROPEAN PATENT SPECIFICATION

(11) **EP 0 288 234 B1**
(45) Date of publication and mention of the grant of the patent: **12.01.1994**
(21) Application number: 88303493.6
(22) Date of filing: 19.04.1988
(51) Int. Cl.: A61N 1/32, A61N 1/34

(54) **Biological tissue stimulator with time-shared logic driving output timing and high-voltage step-up circuit**
Biologischer Gewebestimulator mit logischem Treiberstufenausgang mit Time-sharing und Spannungsvervielfachungsstufe
Stimulateur de tissus biologiques à sortie logique de commande en temps partagé et circuit élévateur de tension

(30) Priority: 24.04.1987 US 42166
(43) Date of publication of application: 26.10.1988
(73) Proprietor: MINNESOTA MINING AND MANUFACTURING COMPANY, St. Paul, Minnesota 55133-3427 (US)
(72) Inventor: Dufresne, Joel Rivar, P.O. Box 33427 St. Paul Minnesota 55133 (US); Dieken, Alan Paul, P.O. Box 33427 St. Paul Minnesota 55133 (US)
(74) Representative: Baillie, Iain Cameron

(56) References cited:
- EP-A- 0 138 347
- EP-A- 0 173 419
- EP-A- 0 246 064
- EP-A- 0 288 260
- FR-A- 2 528 708
- Modern Electronic Circuits Reference Manual; Markus John, 1980; pages 966 and 968

## Description

### Technical Field

The present invention relates generally to biological tissue stimulators and relates more particularly to biological tissue stimulators having high-voltage output circuits.

### Background Art

Biological tissue stimulators are known to be medically useful. In one example, a transcutaneous electrical nerve stimulators (TENS) are utilized to mask pain signals in a human body before they reach the brain giving the subject apparent relief from pain. In such TENS devices, electrical pulses, usually current pulses of a selected rate, amplitude, pulse width and duty cycle, are delivered to the skin of the subject by one or more electrodes. The timing characteristic of the delivered pulses may be predetermined, as for example, by the prescribing physician and/or may be individually selected or controlled by switches available to be operated by the subject. Additionally, individual parameters, or even entire pulse programs, can be varied in a predetermined or random basis by the TENS device itself.

Another example of useful biological tissue stimulators are neuromuscular stimulators (NMS) which can be utilized to electrically stimulate muscle activity of a patient. In such neuromuscular stimulators electrical pulses, again probably current pulses of a carefully controlled rate, amplitude, pulse width and sequence are delivered by electrodes to a site or sites near the muscle to be stimulated in order to activate or contract the muscle. The initiation and control of such sequence of pulses may be patient controlled.

In both of these biological tissue stimulators, an output stage supplies electrical pulses having certain timing and amplitude characteristics. The amplitude of these electrical current pulses is usually of a substantial, e.g., 10-100 milliamperes, current level. Such current levels are achievable by means of a high-voltage power supply, e.g., 40 to 100 volts. The logic circuits necessary for supplying the timing and amplitude information are typically powered by a low-voltage level, e.g., 2.7 to 5 volts. Thus, in order to both service the high-voltage output circuits and the low-voltage logic circuits, at least two separate voltage levels, i.e., sources of electrical power or energy, must be maintained.

Since the biological tissue stimulators are designed to be utilized with a human subject, small size and independence from external power sources are strongly desired. Small batteries are commonly utilized as a source of electrical energy. Since sources of electrical energy at several voltage levels are required, either batteries of several different voltages are required, or the voltage from one set of batteries must be either stepped down or stepped up to obtain the additional voltage levels. The smaller size requirements and operating cost constraints for biological tissue stimulator mitigates against the use of batteries with a significantly higher voltage, e.g., 22.5 to 90 volts. However, the use of batteries of a lower voltage level, e.g. 1.2 to 9 volts, necessitates additional logic and control circuitry to achieve the stepped up voltage with appropriate regulation. These additional components also mitigate against small size due to their number and the increased size of the energy source (batteries) required because of the additional power they consume.

EP-A-0,246,064 having a priority date of 14 May 1986 and published 19 November 1987 discloses a programmable defibrillator comprising a rechargeable battery coupled via a high voltage regulator to a high voltage capacitor. Output switches provide output defibrillation pulses under the control of a microprocessor which also controls the regulator.

FR-A-2,528,708 discloses an implantable apparatus for correcting abnormal cardiac activity by low energy shocks consisting of a supply circuit feeding an energy store. A switch controlled by a programmable logic unit, provides pulses to an output electrode, and the programmable logic unit also controls the supply circuit.

EP-A-0,173,419 discloses a transcutaneous nerve stimulator using a common microprocessor for pulse production and parameter display.

### Disclosure of Invention

The present invention solves these problems by providing a biological tissue stimulator which requires in its most elementary forms, only a low-voltage energy source, e.g., an approximately 3-volt battery, and a step-up voltage circuit to create a high-voltage level energy source. The stimulator has a single logic circuit, e.g., a microprocessor, which is time-shared between supplying timing and amplitude signals to an output stage and supplying a switching signal to the high-voltage step-up circuit.

Thus, a biological tissue stimulator is provided having the advantageous utilization of a single low-voltage energy source, together with the time-shared utilization of an otherwise necessary logic circuitry to drive a step-up voltage circuit. The basic clocking rate and available "time-slice" is determined largely by the timing requirements of the output pulses. Since the duty cycle of stimulation pulse trains is generally low, however, considerable time is available for the logic circuitry to perform the additional task of driving the step-up voltage circuit. Thus, this function is available for "free", i.e., without additional components. Thus, a biological tissue stimulator is achieved with small size and great ease of portability.

In a preferred embodiment, the biological tissue stimulator first determines and gates the appropriate timing and amplitude characteristics to the output circuit. Following this, the stimulator then drives the step-up voltage circuit to generate the higher voltage level required. After a time, the stimulator returns to again gate timing and amplitude information to the output circuit, either the same output channel, or another, e.g., second, output channel. Following this, the stimulator again drives the step-up voltage circuit. Subsequently, the stimulator again gates timing and amplitude characteristics to an output channel, and so on. A storage mechanism stores the generated high-voltage energy until it can be used by an output stage. A voltage regulator circuit determines when the high-voltage storage level has reached a maximum, or a predetermined threshhold, at which point the stimulator then goes to an idle state to conserve power while awaiting the next required output stage gating.

A biological tissue stimulator constructed in this manner achieves significant economies of circuit component count and therefore of size, weight, power and cost. The biological tissue stimulator also minimizes the number of voltage source levels required and therefore minimizes size, weight, power and cost. Further, the biological tissue stimulator achieves economies of power consumption, thus, increasing the operating time between battery changes and decreasing the size required for the battery system.

According to the present invention there is provided a biological tissue stimulator, having a low-voltage direct current power source; a storage means for storing a voltage derived from said power source; and an output circuit means operatively coupled to said storage means for providing a plurality of biological tissue stimulation pulses; characterised by a step-up voltage means comprising a switched inductor circuit being operatively coupled to said low-voltage direct current power source for converting said low-voltage direct current power source to a high-voltage output; and
said storage means being coupled to said high voltage output for storing the voltage of said high voltage output by having timing means powered by said low-voltage direct current power source and coupled to said output circuit for supplying a plurality of control signals to said output circuit means for controlling the timing of said biological tissue stimulation pulses and supplying a plurality of switching signals to said switched inductor circuit for driving said switched inductor, said plurality of control signals alternating with said plurality of switching signals. In a preferred embodiment, the switched inductor circuit has an inductor coupled in series with the low-voltage power source and a switching element coupled in series with the inductor for selectively coupling the inductor to electrical ground in response to the switching signal from the timing mechanism. A rectification mechanism is coupled between the inductor in the switching element for allowing that current which flows through the rectification mechanism to flow only from the inductor. A connection mechanism couples the rectification mechanism and the high-voltage storage mechanism together.

In an alternative embodiment of the present invention, a biological tissue stimulator has a low-voltage power source and a step-up voltage mechanism coupled to the low-voltage power source for converting the low-voltage power source to a high-voltage output in response to switching signals. An output circuit is coupled to the step-up voltage mechanism for generating a biological tissue stimulation current pulse from timing signals. A mode control mechanism is coupled to the step-up voltage mechanism and the output circuit for controlling the biological tissue stimulator alternately in a pulse output mode as needed to supply the timing signals to the output circuit, and in a high-voltage generation mode when not needed in the pulse output mode to supply the switching signals to the step-up voltage mechanism. In a preferred embodiment, the biological tissue stimulator further has an idle mode to conserve the low-voltage power source when the biological stimulator is not needed in either the pulse output mode nor the high-voltage generation mode.

### Brief Description of Drawings

The foregoing advantages, operation and construction of the present invention will become more readily apparent from reference to the following description and accompanying drawings in which:
Figure 1 is a block diagram of a biological tissue stimulator of the present invention showing time-shared programmable logic;
Figure 2 is a block diagram of a biological tissue stimulator of the present invention showing detail of the programmable logic;
Figure 3 is a detailed circuit diagram of the high-voltage step-up circuit of the biological tissue stimulator of the present invention;
Figure 4 is a detailed circuit diagram of an output stage of the biological tissue stimulator of the present invention;
Figure 5 illustrates a mode diagram of a biological tissue stimulator of the present invention;
Figure 6 illustrates a mode diagram of an alternative embodiment of the biological tissue stimulator of the present invention;
Figure 7 illustrates timing diagrams of outputs of the programmable logic circuit of the biological stimulator of the present invention; and
Figure 8 illustrates timing diagrams of timing signals driving the step-up voltage circuit.

### Detailed Description of the Preferred Embodiments

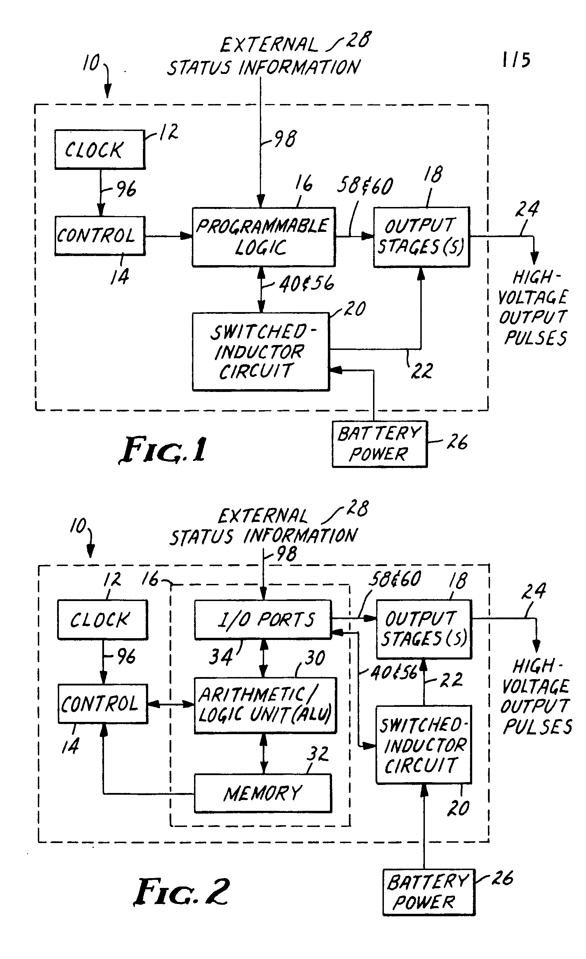

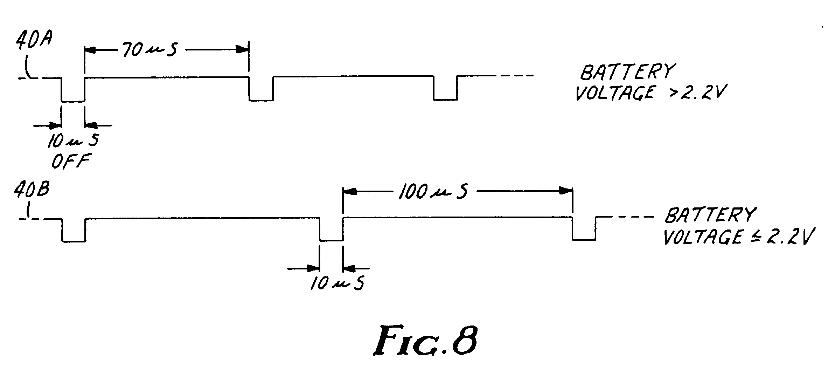

Figures 1 and 2 illustrate block diagrams of the biological tissue stimulator 10. A clock 12 supplies basic timing signals to a control circuit 14 which controls programmable logic block 16. Programmable logic block 16 operates on a time-shared basis between supplying timing and amplitude information (58 & 60) to output stage 18 and a switching signal 40 to switched-inductor circuit 20. The high-voltage output signal 22 is also utilized by output stage 18 to supply high-voltage output pulses 24 for use by the patient or subject receiving biological tissue stimulation pulses from the biological tissue stimulator 10. A low-voltage source of battery power 26 is shown coupled to the switched inductor circuit 20. Programmable logic circuit 16 also receives external status information 28 which is expected to be in the form of operator controlled amplitude or mode select switches. In Figure 2, programmable logic circuit 16 is shown as consisting of conventional arithmetic logic unit (ALU) 30, appropriate memory storage elements 32, and input-output ports 34. Such blocks and circuits are conventional in nature and the construction and operation of programmable logic block 16 in conjunction with control 14 and clock 12 will become readily apparent later in the discussion of Figures 7 and 8 illustrating timing diagrams.

In the biological tissue stimulator 10, the single programmable logic block 16 is time-shared between the output timing and amplitude considerations and directly driving the switched inductor circuit 20. Since only a single low-voltage level battery source 26 is required, battery source 26 could supply not only the source of low-voltage to switched inductor circuit 20, to be converted to a high-voltage level, but could also advantageously supply the low-voltage level power required by the logic elements contained within clock 12, control 14, programmable logic 16, and a portion of output stage 18. In the preferred embodiment, a single source of battery power 26 supplies both of these functions. Alternatively, separate battery sources 26 could supply (1) the switched inductor circuit 20 and (2) the power for the low level logic circuits. However, it is noted that only a single low-voltage level power source is required whether it comes physically from one battery element or a plurality of battery elements. Of course, it is also possible to power all logic elements from a separate, regulated, low-voltage power supply connected to the basic low-voltage power source, i.e. the batteries.

Since programmable logic block 16 is time-shared between output stage 18 and switched-inductor circuit 20, programmable logic block 16 will at one instant of time by supplying the timing and amplitude characteristics of the output pulses 24 to the output stage 18 and at another instant of time driving switched inductor circuit 20 to create the high-voltage output 22. Since the basic rate of signals from clock 12 is at least partially determined by the timing requirements of the short duty-cycle, high-voltage stimulation pulses 24, "time" is available for programmable logic block 16 to perform the additional task of driving the switched-inductor circuit 20. Thus, the task of driving the switched inductor circuit 20 is achieved essentially for "free".

Although a single output stage 18 is illustrated in Figures 1 and 2, it is to be recognized and understood that the high-voltage level 22 from switched-inductor circuit 20 could be supplied to a plurality of output stages 18 and that programmable logic block 16 could be additionally time-shared to supply timing and amplitude characteristics to the high-voltage output pulses 24 to be delivered by the additional output stages 18.

Switched inductor circuit 20 is shown in a detailed schematic in Figure 3. An inductor 36 is connected to one end of the low-voltage power source, battery, 26. In a preferred embodiment, inductor 36 is a one millihenry inductor and battery 26 are two size "AAA" batteries in series supplying a voltage level of 2-3 volts. The opposite end of inductor 36 is coupled through a high-voltage gate, or switching circuit, 38 coupled to electrical ground. Gate 38 is driven by switching signal 40 supplied from programmable logic block 16 (in Figures 1 and 2). The end of inductor 36 which is coupled to gate 38 is also coupled to the anode of diode 42 to rectify the signal received from inductor 36 and to assure that current is only retrieved from inductor 36 and not returned to it. Essentially, potential energy from battery 26 is transformed into magnetic energy within the inductor 36 when gate 38 is momentarily opened, i.e., transistor gate 38 is conducting, by the programmable logic block 16. This magnetic energy is subsequently transformed into potential energy in capacitor 44 when gate 38 is closed, i.e., transistor gate 38 is not conducting. The current passes through diode 42 and is stored in capacitor 44, operating as a storage element, until it can be subsequently utilized by the output stage 18. The basic process of opening and closing gate 38, as controlled by switching signal 40, is repeated by the programmable logic block until the appropriate high-voltage signal 22 level is reached. A back biased zener diode 46 provides basic over-voltage protection. In a preferred embodiment zener diode 46 is an approximately 62 volt zener diode. High-voltage signal 22 is maintained at approximately 50-55 volts by a voltage regulation system consisting of resistors 48 and 50 forming a voltage divider coupled to one input of a comparator 52 whose other input terminal is a stable reference voltage source 54, which in a preferred embodiment is approximately 1.7 volts. The output 56 of comparator 52 is fed back to programmable logic block 16 for use by the programmable logic block 16 for monitoring the status of the high-voltage circuit 20 and for providing feedback so that the programmable logic unit 16 may appropriately control the mode of operation of the biological tissue stimultor 10.

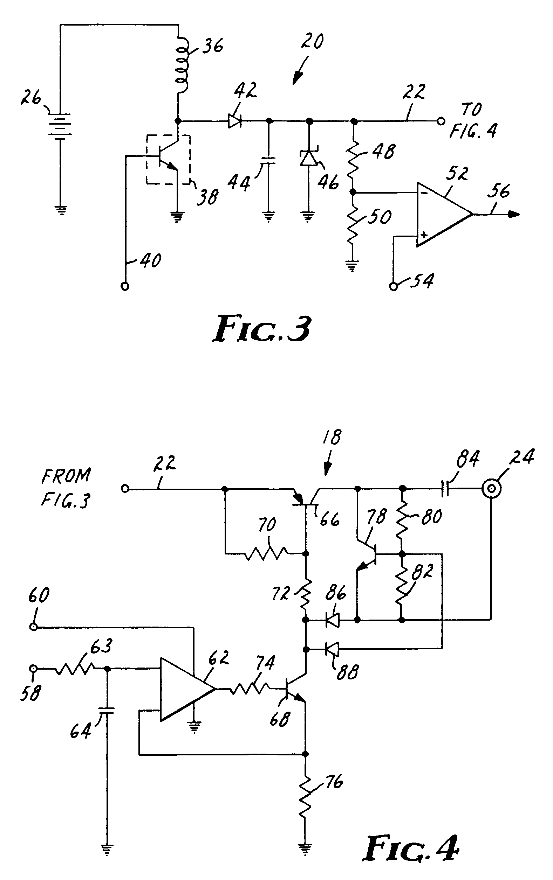

A detailed circuit diagram of the output stage 18 of a preferred embodiment of the present invention is illustrated in Figure 4. Amplitude signal 58, received from the programmable logic unit 16, is a pulse train with a fixed pulse repetition rate. The pulse width of each individual pulse is dependent upon the amplitude desired for the high-voltage output pulse 24 which are to be provided by the output stage 18. Output stage 18 also receives timing signal 60 from the programmable logic block 16. Timing signal 60 is activated to trigger operational amplifier 62 to initiate a high-voltage output pulse 24 from the output stage 18. Capacitor 64 and resistor 63 form a low-pass filter which essentially recovers the DC component of the variable width pulse train from amplitude signal 58. Output stage 18 also receives high-voltage signal 22 from the switched inductor circuit 20. Transistors 66, 68 and resistors 70, 72, 74 and 76 transform the high-voltage signal 22 into a current pulse to be supplied as high-voltage output pulse 24. Transistor 78, resistors 80 and 82, and capacitor 84 ensure a zero net DC component for the output current pulse train 24. Diodes 86 and 88 permit transistor 78 to conduct only when transistor 68 is not conducting.

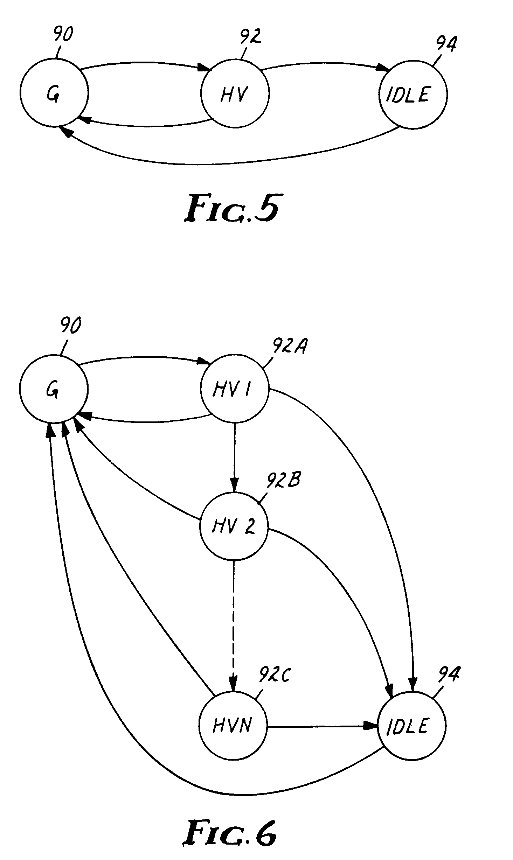

Figure 5 illustrates one preferred embodiment of the modes of operation of the biological tissue stimulator 10 of the present invention. Generally, in Figure 5, the biological tissue stimulator is in state 90 providing amplitude and timing characteristics 58 and 60 to one of the output stages 18. When that task is completed, the biological tissue stimulator then moves to state 92 to supply switching signals 40 to a switched-inductor circuit 20 operating as a high-voltage step-up circuit which creates a high-voltage level 22 for subsequent use by the output stages 18. When finished with the task of maintaining the high-voltage supply 22, biological tissue stimulator 10 may either (1) return immediately or after a predetermined amount of time has transpired before completing stepping up the high-voltage supply level 22, or (2) to state 90 to again supply amplitude and timing characteristics to one or more of the output stages 18. When the voltage level of the high-voltage supply 22 reaches the correct value, the biological tissue stimulator may then move to state 94 to "idle" in order to conserve energy from the low-voltage power source 26.

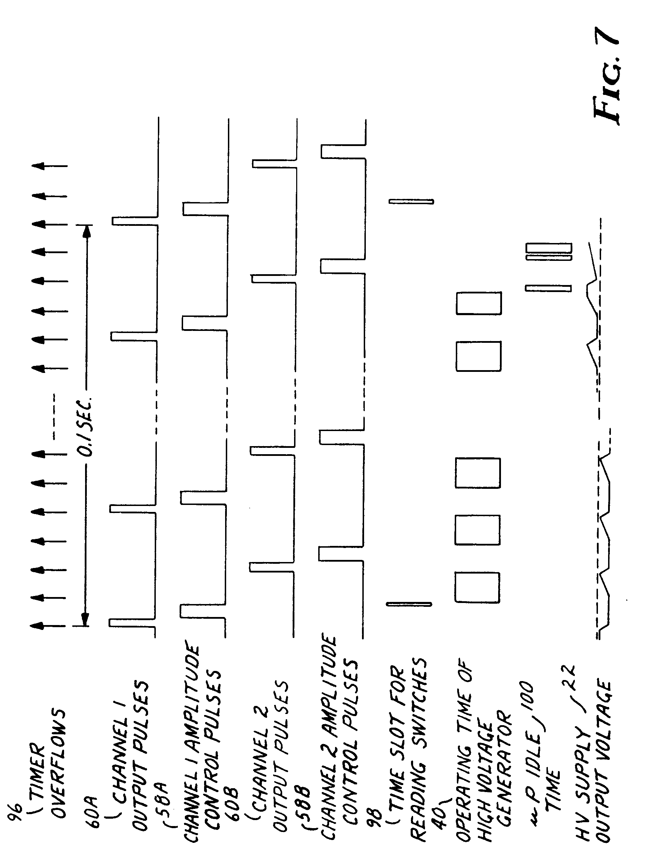

Figure 6 shows a variation of the state diagram in Figure 5. Figure 6 is an alternative in which a single state 90, used to supply timing and amplitude characteristics to output stages 18 serves a plurality of high-voltage step-up circuits 20 and, hence, must individually sequentially move through states 92A, 92B to 92C to complete supplying switching signals 40 to all of the available high-voltage switched inductor circuits 20 included in the biological tissue stimulator 10. If any time is available, again, idle state 94 is reached.

Alternatively, to the mode of operation illustrated in Figure 6, a single switched inductor circuit 20 may serve a plurality of output stages 18. In this case, only one switched inductor circuit 20 is supplied with switching signals 40 while the plurality of output stages 18 are supplied timing and amplitude information again on a time-shared basis. This can be more readily illustrated from the detailed timing diagram illustrated in Figure 7. Figure 7 illustrates a plurality of timer overflows 96 which would be supplied from clock 12 and which represents the basic timing intervals available to programmable logic block 16. The timing diagram in Figure 7 presumes that there are two output stages 18 given a nomenclature of channel 1 and channel 2 and a single switched-inductor circuit 20. During the first interval between timer overflows 96, channel 1 output pulse 60A is supplied immediately followed by channel 1 amplitude signal 58A. The time-averaged pulse width of amplitude signal 58A is variable and, in the output stage 18, determines the amplitude of the delivered output current pulse 24. In a preferred embodiment, immediately after supplying the channel 1 amplitude signal 58A, programmable logic block 16 will read external status information 28 as indicated by signal 98. If any time remains in the first timer overflow 96 interval, programmable logic block 16 will then start supplying switching signal 40 to switched inductor circuit 20. In a preferred embodiment, the entire second timer overflow 96 interval, if required, is used to supply switching signal 40 to the switched inductor circuit 20. During the third timer overflow 96 interval, channel 2 timing signal 60B and channel 2 amplitude signal 58B is supplied to the channel 2 output stage 18. Since in a preferred embodiment external status information is only read on a longer interval, programmable logic unit 16 will then move directly to supply switching signal 40 to switched inductor circuit 20. The fourth timer overflow interval 96 is similar to the second timer overflow 96 interval in that the entire time period, if required, is spent supplying switching signal 40 to switched inductor circuit 20. In this timing diagram, it is presumed that the high-voltage level signal 22 is lower than desired and a maximum amount of time is spent supplying switching signals 40 to build high-voltage signal level 22 up to the desired value. Further timer overflow 96 intervals are similar to those preceding until the high-voltage level supply 22 reaches its maximum level, or its predetermined threshhold level. At this time, programmable logic block 16 instead of continuing to supply switching signals 40 to the switched inductor circuit 20 will instead go to the idle state as evidenced by idle blocks 100 since this time is not required to maintain the high-voltage supply level 22. Also, on a periodic basis, another time slot 98 is reserved for reading external status information 28.

Figure 8 illustrates a preferred timing diagram of the switching signal 40 to be supplied to switched inductor circuit 20. The exact period of time that gate 38 is to be opened and closed ideally should depend upon the magnitude of the inductance of inductor 36, desired efficiency levels (reflecting power losses in major components), output current requirements and, most significantly, the voltage supplied by battery 26. Since battery voltage is not an operational constant, the timing diagrams illustrated in Figure 8 adjust depending upon the voltage level. Generally, as the batteries 26 discharge, their voltage level will decrease. In general, gate 38 should not be open past the point of inductor 36 saturation since power losses will increase greatly at this point. However, gate 38 should still be open long enough to build sufficient energy storage in the inductor. Magnetic energy increases with the square of the peak current through the inductor. The voltage supplied from batteries 26 is approximately equal to the value of the inductance in the inductor 36 times the quantity, the change in current divided by the change in time. In order to maintain the change in current constant as the battery voltage decreases, the change in time, or the amount of time the gate 38 is closed, must increase. It is generally important to keep the peak current rather constant in order to maintain the power capacity of the high-voltage signal level 22 while limiting the time the switched inductor circuit must be operated. In other words, the "time-slice" required from the programmable logic block 16 remains well-bounded despite changes in battery 26 voltage. The time that gate 38 is open is preferred to be a function of the battery 26 voltage. This is illustrated in the timing diagrams of switching signal 40 illustrated in Figure 8. In a preferred embodiment, two discrete timing signals are supplied. Switching signal 40A is supplied when the battery voltage is greater than 2.2 volts. The timing diagram of switching signal 40A indicates that switching signal 40A is "on" (causing gate 38 to conduct) for 70 microseconds and "off" (causing gate 38 not to conduct) for 10 microseconds. When the battery voltage drops below 2.2 volts, switching signal 40B is then utilized. In order to maintain proper current levels, the "on" time of switching signal 40 is increased to 100 microseconds. However the "off" time remains constant at 10 microseconds. The "on" time of switching signal 40 is varied in order to get peak operation from inductor 36 while the "off" time of switching signal 40 is kept constant since this amount of time is only necessary to get the current out of the inductor into the capacitor 44 to store the current as a voltage available as high-voltage signal level 22.

## Claims

1. A biological tissue stimulator, having a low-voltage direct current power source (26); a storage means (44) for storing a voltage derived from said power source; and an output circuit means (18) operatively coupled to said storage means (44) for providing a plurality of biological tissue stimulation pulses; characterised by a step-up voltage means comprising a switched inductor circuit (20) being operatively coupled to said low-voltage direct current power source (26) for converting said low-voltage direct current power source to a high-voltage output; and
said storage means (44) being coupled to said high voltage output (24) for storing the voltage of said high voltage output by having timing means (16) powered by said low-voltage direct current power source (26) and coupled to said output circuit (18) for supplying a plurality of control signals (58, 60) to said output circuit means for controlling the timing of said biological tissue stimulation pulses and supplying a plurality of switching signals (40, 56) to said switched inductor circuit for driving said switched inductor, said plurality of control signals alternating with said plurality of switching signals.

2. A biological tissue stimulator as in claim 1 further having regulating means (48, 50, 52) operatively coupled to said storage means (44) and said timing means (16) for disabling said switching signals to said switched inductor circuit when said high-voltage means has achieved a predetermined voltage level.

3. A biological tissue stimulator as in claim 1 wherein said switched inductor circuit (20) converts said low-voltage direct current power to said high voltage output in response to switching signals (40); wherein said output circuit means generates said plurality of biological tissue stimulation current pulses from timing signals (58, 60); and wherein said timing means (16) operates alternately in a pulse output mode, as needed to supply said timing signals to said output circuit means (18), and in a high-voltage generation mode, to supply said switching signals to said switched inductor circuit (20).

4. A biological tissue stimulator as in claim 3 wherein said timing means (16) has an idle mode to conserve said low-voltage power when not needed in either said pulse output mode nor in said high-voltage generation mode.

5. A biological tissue stimulator as in claim 4 further having regulating means (48, 50, 52) operatively coupled to said storage means (44) and said timing means (16) for disabling said switching signals to said switched inductor circuit when said storage means has achieved a predetermined voltage level.

6. A biological tissue stimulator as in claims 1 or 3 wherein said switched-inductor circuit has an inductor (36) coupled in series with said low-voltage direct current power source (26); a switching element (38) coupled in series with said inductor for selectively coupling said inductor to electrical ground in response to said switching signal from said timing means; rectification means (42) coupled between said inductor (36) and said switching element for allowing that current which flows through said rectification means to flow only from said inductor; and connection means for coupling together said rectification means (42) and said storage means (44).

7. A biological tissue stimulator as in claim 6 in which the duty cycle of said switching signal coupling said inductor to electrical ground is a function of the voltage level of said low-voltage power source.

## Patentansprüche

1. Biologischer Gewebestimulator mit einer Niederspannungsgleichstromquelle (26); einer Speichereinrichtung (44) zum Speichern einer von der Stromquelle hergeleiteten Spannung; und einer Ausgangsschaltungseinrichtung (18), die funktional mit der Speichereinrichtung (44) gekoppelt ist, um eine Vielzahl von biologischen Gewebestimulationsimpulsen abzusetzen; dadurch gekennzeichnet, daß eine Spannungsvervielfachungseinrichtung eine Induktorschaltung (20) umfaßt, die funktional mit der Niederspannungsgleichstromquelle (26) gekoppelt ist, um die Niederspannungsgleichstromquelle auf einen Hochspannungsausgang umzuschalten; und
daß die Speichereinrichtung (44) mit dem Hochspannungsausgang (24) gekoppelt ist, um die Spannung des Hochspannungsausgangs zu speichern, indem eine Zeitgebereinrichtung (16) vorgesehen ist, die von der Niederspannungsgleichstromquelle (26) versorgt wird und mit der Ausgangsschaltung (18) gekoppelt ist, um eine Vielzahl von Steuersignalen (58, 60) an die Ausgangsschaltungseinrichtung abzusetzen, die den zeitlichen Ablauf der biologischen Gewebestimulationsimpulse steuern, und um eine Vielzahl von Schaltsignalen (40, 56) an die Induktorschaltung abzusetzen, um die Induktorschaltung zu betätigen, wobei die Vielzahl von Steuersignalen mit der Vielzahl von Schaltsignalen abwechselt.

2. Biologischer Gewebestimulator nach Anspruch 1, ferner umfassend eine Regelungseinrichtung (48, 50, 52), die funktional mit der Speichereinrichtung (44) und der Zeitgebereinrichtung (16) verbunden ist, um die an die Induktorschaltung abgesetzten Schaltsignale zu unterbrechen, wenn die Hochspannungseinrichtung einen vorbestimmten Spannungspegel erreicht hat.

3. Biologischer Gewebestimulator nach Anspruch 1, bei dem die Induktorschaltung (20) die Niederspannungsgleichstromquelle in Abhängigkeit von den Schaltsignalen (40) auf den Hochspannungsausgang umschaltet; wobei die Ausgangsschaltungseinrichtung die Vielzahl von biologischen Gewebestimulationsimpulsen aus den Zeittaktsignalen (58, 60) generiert; und wobei die Zeitgebereinrichtung (16) abwechselnd in einem Impulsausgabemodus, der benötigt wird, um die Zeittaktsignale an die Ausgangsschaltungseinrichtung (18) zu liefern, und in einem Hochspannungsgenerierungsmodus arbeitet, bei dem die Schaltsignale zu der Induktorschaltung (20) geliefert werden.

4. Biologischer Gewebestimulator nach Anspruch 3, bei dem die Zeitgebereinrichtung (16) einen Ruhezustand besitzt, um den Strom niedriger Spannung zu konservieren, wenn er weder im Impulsausgabemodus noch im Hochspannungsgenerierungsmodus gebraucht wird.

5. Biologischer Gewebestimulator nach Anspruch 4, des weiteren mit einer Regelungseinrichtung (48, 50, 52), die funktional mit der Speichereinrichtung (44) und der Zeitgebereinrichtung (16) gekoppelt ist, um die zu der Induktorschaltung gelieferten Schaltsignale zu unterbrechen, wenn die Speichereinrichtung einen vorbestimmten Spannungspegel erreicht hat.

6. Biologischer Gewebestimulator nach Anspruch 1 oder 3, bei dem die Induktorschaltung einen Induktor (36) besitzt, der mit der Niederspannungsgleichstromquelle (26) in Reihe geschaltet ist; ein Schaltelement (38), das mit dem Induktor in Reihe geschaltet ist, um den Induktor entsprechend dem Schaltsignal von der Zeitgebereinrichtung wahlweise an Erde zu legen; eine Gleichrichtereinrichtung (42), die zwischen dem Induktor (36) und dem Schaltelement geschaltet ist, damit Strom, der durch die Gleichrichtereinrichtung fließt, nur von dem Induktor kommen kann; und eine Verbindungseinrichtung, die die Gleichrichtereinrichtung (42) und die Speichereinrichtung (44) miteinander koppelt.

7. Biologischer Gewebestimulator nach Anspruch 6, bei dem der Arbeitszyklus des Schaltsignals, welches den Induktor an Erde legt, eine Funktion des Spannungspegels der Niederspannungsstromquelle ist.

## Revendications

1. Stimulateur de tissus biologiques, comprenant une source de courant continu à faible tension (26), des moyens d'accumulation (44), servant à accumuler une tension fournie par la source de courant, et un circuit de sortie (18) connecté fonctionnellement aux moyens d'accumulation (44) de façon à fournir plusieurs impulsions de stimulation des tissus biologiques, caractérisé par des moyens élévateurs de tension comprenant un circuit à inductance commutée (20) qui est connecté fonctionnellement à la source de courant continu à faible tension (26) de façon à convertir cette source de courant continu à faible tension en une sortie à tension élevée, tandis que les moyens d'accumulation (44) sont connectés à la sortie à tension élevée (24) de façon à accumuler la tension de cette sortie à tension élevée, du fait de la présence de moyens de variation dans le temps (16) alimentés en énergie par la source de courant continu à faible tension (26) et connectés au circuit de sortie (18) de façon à fournir de multiples signaux de commande (58, 60) au circuit de sortie, en vue de commander la variation dans le temps des impulsions de stimulation des tissus biologiques, et à fournir de multiples signaux de commutation (40, 56) au circuit à inductance commutée, en vue d'attaquer l'inductance commutée, les multiples signaux de commande alternant avec les multiples signaux de commutation.

2. Stimulateur de tissus biologiques suivant la revendication 1, comportant en outre des moyens de régulation (48, 50, 52) connectés fonctionnellement aux moyens d'accumulation (44) et aux moyens de variation dans le temps (16) de façon à supprimer lesdits signaux de commutation, envoyés au circuit à inductance commutée, lorsque les moyens à tension élevée ont atteint un niveau préfixé de tension.

3. Stimulateur de tissus biologiques suivant la revendication 1, dans lequel le circuit à inductance commutée (20) convertit ladite énergie en courant continu et faible tension pour donner ladite sortie à tension élevée sous l'action de signaux de commutation (40), tandis que le circuit de sortie produit les multiples impulsions de courant de stimulation des tissus biologiques à partir de signaux de variation dans le temps (58, 60) et dans lequel les moyens de variation dans le temps (16) fonctionnent alternativement dans un mode de sortie d'impulsions, tel que nécessaire pour fournir les signaux de variation dans le temps au circuit de sortie (18), et dans un mode de production de tension élevée, lorsqu'il n'est pas nécessaire dans le mode de sortie d'impulsions, de façon à fournir les signaux de commutation au circuit à inductance commutée (20).

4. Stimulateur de tissus biologiques suivant la revendication 3, dans lequel les moyens de variation dans le temps (16) comportent un mode de repos permettant de conserver l'énergie à faible tension lorsque le stimulateur biologique n'est nécessaire ni dans le mode de sortie d'impulsions, ni dans le mode de production de tension élevée.

5. Stimulateur de tissus biologiques suivant la revendication 4, comportant en outre des moyens de régulation (48, 50, 52) connectés fonctionnellement aux moyens d'accumulation (44) et aux moyens de variation dans le temps (16) de façon à supprimer lesdits signaux de commutation envoyés au circuit à inductance commutée, lorsque les moyens d'accumulation ont atteint un niveau préfixé de tension.

6. Stimulateur de tissus biologiques suivant l'une des revendications 1 et 3, dans lequel le circuit à inductance commutée comprend une inductance (36), connectée en série avec la source de courant continu à faible tension (26), un élément de commutation (38), connecté en série à l'inductance de façon à connecter de manière sélective l'inductance à la masse sous l'action du signal de commutation provenant des moyens de variation dans le temps, des moyens de redressement (42), connectés entre l'inductance (36) et l'élément de commutation de façon à permettre au courant qui passe dans les moyens de redressement de ne passer qu'à partir de l'inductance, et des moyens de connexion servant à connecter entre eux les moyens de redressement (42) et les moyens d'accumulation (44).

7. Stimulateur de tissus biologiques suivant la revendication 6, dans lequel le rapport cyclique du signal de commutation connectant l'inductance à la masse est une fonction du niveau de tension de la source de courant à faible tension.
